# EUROPÄISCHE PATENTSCHRIFT

(11) **EP 1 505 126 B1**
(45) Veröffentlichungstag und Bekanntmachung des Hinweises auf die Patenterteilung: **19.12.2012**
(21) Anmeldenummer: 04013566.7
(22) Anmeldetag: 09.06.2004
(51) Int. Cl.: C09C 1/56

(54) **Organische Thiocyanatgruppen enthaltender Russ**
Carbon black comprising organic thiocyanate groups
Noire de carbonne contenant des groupes organiques de thiocyanates

(30) Priorität: 08.08.2003 DE 10336575
(43) Veröffentlichungstag der Anmeldung: 09.02.2005
(73) Patentinhaber: Evonik Carbon Black GmbH, 63457 Hanau (DE)
(72) Erfinder: Niedermeier, Werner, Dr., 50321 Brühl (DE); Fröhlich, Joachim, Dr., 53332 Bornheim (DE); Bergemann, Klaus, Dr., 50170 Kerpen-sindorf (DE); Lüthge, Thomas, Dr., 63452 Hanau (DE); Fanghänel, Egon, Prof., 06114 Halle (DE); Knackfuss, Bernd, Dr., 04229 Leipzig (DE)
(74) Vertreter: Polypatent

(56) Entgegenhaltungen:
- EP-A1- 1 136 526
- WO-A1-92/13983
- WO-A1-98/42778
- US-A1- 2002 096 089

## Beschreibung

Die Erfindung betrifft eine rußhaltige Kautschukmischung und ein Verfahren zu deren Herstellung.

Aus DE 10012783 sind Ruße mit organischen Gruppen bekannt, wobei die organische Gruppe mindestens eine substituierte C-C-Einfach- oder Doppelbindung enthält, über die zwei Kohlenstoffatome der C-C-Einfach- oder Doppelbindung mit dem Ruß verbunden ist und mindestens ein Kohlenstoffatom der C-C-Einfach- oder Doppelbindung mindestens einen aktivierenden Substituenten enthält.

Aus EP 0569503 ist ein Verfahren zur Oberflächenmodifizierung von kohlenstoffhaltigem Material mit aromatischen Gruppen durch elektrochemische Reduktion eines Diazoniumsalzes bekannt.

Weiterhin ist bekannt, Ruß mit organischen Gruppen zu versehen, indem man die organischen Gruppen über eine Diazotierung mit dem kohlenstoffhaltigen Material verknüpft (WO 96/18690) oder die organischen Gruppen mittels Umsetzungen mit Radikalbildnern (Ohkita K., Tsubokawa N., Saitoh E., Carbon 16 (1978) 41), DE 10012784.3) an den Ruß bindet.

EP 1 136 526 A offenbart Ruße mit organischen Gruppen, wobei die organischen Gruppen über mindestens eine Sulfid-und/oder Polysulfidbrücke mit dem Ruß verbunden sind und ein mit Thiocyanat funktionalisiertes Alkyl mit umfassen. Diese Ruße können als Füllstoff, UV-Stabilisator, Leitfähigkeitsruß und Pigment verwendet werden.

Aus US 2002/0096089 sind Ruße mit organischen Gruppen bekannt, wobei die organischen Gruppen über ein oder zwei C-Atome der C-C-Einfachbindung oder Doppelbindung mit dem Ruß verbunden sind und eine Aktivierungsgruppe enthalten.

WO 92/13983 offenbart die Modifizierung von kohlenstoffhaltigem Material durch elektrochemische Reduktion von Diazoniumsalz.

WO 98/42778 offenbart Kautschukmischungen enthaltend Rußpartikel, die metallhaltige Phasen enthalten. Die Kautschukmischung kann beispielsweise Silane unter anderem Thiocyanatopropyl-Triethoxy-Silan enthalten.

Ein Nachteil der bekannten Ruße ist, daß sich bei Verwendung von oberflächenmodifizierten Rußen in Kautschukmischungen die Hysterese (korreliert mit dem Rollwiderstand) und/oder die dynamische Steifigkeit (korreliert mit Handlingeigenschaften) verschlechtert.

Aufgabe der vorliegenden Erfindung ist es eine rußhaltige Kautschukmischung zur Verfügung zu stellen, die eine höhere oder gleiche dynamische Steifigkeit (E* 60°C) bei gleichzeitig reduzierter Hysterese (tan δ 60°C) aufweist.

Gegenstand der Erfindung ist eine Kautschukmischung, dadurch gekennzeichnet, dass diese einen Kautschuk oder eine Mischung von Kautschuken und einen Ruß mit organischen Gruppen enthält und die organische Gruppe eine N-(4-Thiocyanatalkyl)-succinimid-, N-(4-Thiocyanataryl)-succinimid-, N-(4-Thiocyanatalkyl)-amid- oder N-(4-Thiocyanataryl)-amid- Gruppe ist. Besonders bevorzugt ist die Gruppe eine N-(4-Thiocyanato-phenyl)-succinimid- oder N-(4-Thiocyanato-phenyl)-amid-Gruppe. Die Alkyl-Gruppe kann ein zweibindiger verzweigter oder unverzweigter, gesättigter oder ungesättigter Kohlenwasserstoff mit 1 bis 20 C-Atomen sein. Die Aryl-Gruppe kann eine Phenyl- oder Naphtyl-Gruppe sein.

Der Ruß der erfindungsgemäßen Kautschukmischung kann hergestellt werden, indem man Ruß mit C-C-Doppel- oder Dreifachbindung, die nicht Bestandteil eines aromatischen Systems ist, enthaltenden organischen Verbindungen, deren C-C-Doppel- oder Dreifachbindung durch mindestens einen Substituenten aktiviert ist, umsetzt und die organische Verbindung mindestens eine der oben definierten Gruppen enthält.

Aktivierende Substituenten können Akzeptorsubstituenten sein. Akzeptorsubstituenten können sein -COOR, -CO-R, -CN, -SO₂R, -SO₂OR, -CO-X-CO- mit R = H, -NH-R¹, Alkyl, Aryl oder funktionalisiertes Alkyl oder Aryl, X = O oder N-R¹, R¹ = Alkyl, durch Y funktionalisiertes Alkyl, Polymere, cyclische organische Gruppen, Aryl oder durch Y funktionalisiertes Aryl der Form Ar-Yₙ (n=1-5), Y = -OH, - SH, -SO₃H, -SO₃M, -B(OH)₂, -O(CH₂-CH₂-O)ₙH, -COOH, -NH₂, - NR₂, --N((CH₂-CH₂O)ₙH)₂, CON((CH₂-CH₂-O)ₙH)₂, Trialkoxysilyl, Perfluoralkyl, R¹, -NH₃⁺, -NR₃⁺, -SO₂-NR₂, -NO₂, -Cl, -CO-NR₂, -SS- oder -SCN, M = Metall, beispielsweise Alkali⁺ oder Erdalkali⁺⁺, oder NR²₄⁺ mit R²=H, Alkyl oder Aryl.

Die organische Verbindung enthält eine N-(4-Thiocyanatalkyl)-succinimid-, N-(4-Thiocyanataryl)-succinimid-, N-(4-Thiocyanatalkyl)-amid- oder N-(4-Thiocyanataryl)-amid- Gruppe, besonders bevorzugt eine N-(4-Thiocyanato-phenyl)-succinimid- oder N-(4-Thiocyanatophenyl)-amid-Gruppe.

Die Ruße mit organischen Gruppen für die erfindungsgemäße Kautschukmischung können durch Umsetzung der Ausgangsruße mit N-(4-Thiocyanatophenyl)-maleinsäureimid oder N-(4-Thiocyanato-phenyl)-maleinsäureamid hergestellt werden. Ein möglicher

Reaktionsmechanismus könnte sein:

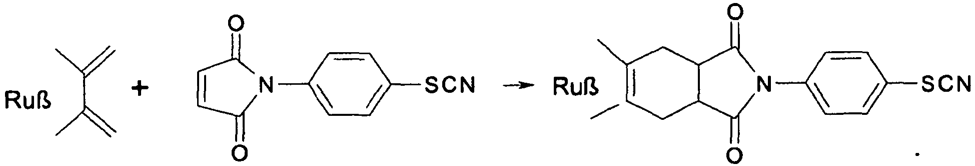

N-(4-Thiocyanato-phenyl)-maleinsäureimid beziehungsweise N-(4-Thiocyanato-phenyl)-maleinsäureamid kann aus Maleinsäureanhydrid und Rhodanoanilin hergestellt werden.

Als Ausgangsruße können Furnaceruß, Gasruß, Channelruß, Flammruß, Thermalruß, Acetylenruß, Plasmaruß, Inversionsruße, bekannt aus DE 195 21 565 und DE 19839925, Si-haltige Ruße, bekannt aus WO 98/45361 oder DE 19613796, oder metallhaltige Ruße, bekannt aus WO 98/42778, Lichtbogenruß und Ruße, die Nebenprodukte chemischer Produktionsprozesse sind, verwendet werden. Der Ruß kann durch vorgelagerte Reaktionen, beispielsweise durch eine Oxidation, aktiviert werden. Es können Farbruße eingesetzt werden. Weitere Ruße können sein: Leitfähigkeitsruß, Ruß zur UV-Stabilisierung, Ruß als Füllstoff in anderen Systemen als Kautschuk, wie zum Beispiel in Bitumen, Kunststoff, Ruß als Reduktionsmittel, in der Metallurgie.

Als Kautschuk können Naturkautschuk und/oder Synthesekautschuke verwendet werden. Bevorzugte Synthesekautschuke sind beispielsweise bei W. Hofmann, Kautschuktechnologie, Genter Verlag, Stuttgart 1980, beschrieben. Sie können unter anderem
- Polybutadien (BR)
- Polyisopren (IR)
- Styrol/Butadien-Copolymerisate mit Styrolgehalten von 1 bis 60, vorzugsweise 5 bis 50 Gew.-% (SBR)
- Isobutylen/Isopren-Copolymerisate (IIR)
- Butadien/Acrylnitril-Copolymere mit Acrylnitrilgehalten von 5 bis 60, vorzugsweise 10 bis 50 Gew.-% (NBR)
- Ethylen/Propylen/Dien-Copolymerisate (EPDM)
sowie Mischungen dieser Kautschuke umfassen.

In einer bevorzugten Ausführungsform können die Kautschuke schwefelvulkanisierbar sein.

Die Kautschukmischungen können 10 bis 150 Gew.-Teile Ruß mit organischen Gruppen enthalten, wobei die Gew.-Teile auf 100 Gew.-Teile Kautschuk bezogen sind.

Die Kautschukmischungen können Organosilane in einer Menge von 0,1 bis 15 Gew.-%, bezogen auf die Menge des eingesetzten Füllstoffs, enthalten. Als Organosilane können Alkylsilane, beispielsweise Octyltrimethoxysilan, Hexadecyltrimethoxysilan, Octadecyltrimethoxysilan, Propyltriethoxysilan oder Octyltriethoxysilan, oder Organopolysulfansilane, beispielsweise Bis-(triethoxysilylpropyl)tetrasulfan oder Bis-(triethoxysilylpropyl)disulfan, verwendet werden.

Die erfindungsgemäßen Kautschukmischungen können weitere bekannte Kautschukhilfsmittel enthalten wie zum Beispiel Vernetzer, Vulkanisationsbeschleuniger, Reaktionsbeschleuniger, -verzögerer, Alterungsschutzmittel, Stabilisatoren, Verarbeitungshilfsmittel, Weichmacher, Wachse, Metalloxide sowie Aktivatoren, wie Triethanolamin, Polyethylenglykol oder Hexantriol.

Die Kautschukhilfsmittel können in üblichen Mengen, die sich unter anderem nach dem Verwendungszweck richten, eingesetzt werden. Übliche Mengen sind zum Beispiel Mengen von 0,1 bis 50 Gew.-%, bezogen auf Kautschuk.

Als Vernetzer können Schwefel oder organische Schwefelspender dienen.

Die erfindungsgemäßen Kautschukmischungen können darüber hinaus Vulkanisationsbeschleuniger enthalten. Beispiele für geeignete Vulkanisationsbeschleuniger sind Mercaptobenzthiazole, Sulfenamide, Guanidine, Thiurame, Dithiocarbamate, Thioharnstoffe und Thiocarbonate. Die Vulkanisationsbeschleuniger und Schwefel können in Mengen von 0,1 bis 10 Gew.-%, bevorzugt 0,1 bis 5 Gew.-%, bezogen auf den eingesetzten Kautschuk, eingesetzt werden.

Ein weiterer Gegenstand der Erfindung ist ein Verfahren zur Herstellung der erfindungsgemäßen Kautschukmischungen, welches dadurch gekennzeichnet ist, daß man den Kautschuk oder die Mischung von Kautschuken und den oben definierten Ruß mit organischen Gruppen in einem Mischaggregat mischt.

Die Abmischung der Kautschuke mit dem oben definierten Ruß mit organischen Gruppen, gegebenenfalls Kautschukhilfsmitteln und Organosilane kann in üblichen Mischaggregaten, wie Walzen, Innenmischern und Mischextrudern, durchgeführt werden. Üblicherweise können solche Kautschukmischungen in Innenmischern hergestellt werden, wobei zunächst in einer oder mehreren aufeinanderfolgenden thermomechanischen Mischstufen die Kautschuke, der Füllstoff, und die Kautschukhilfsmittel bei 100 bis 170°C eingemischt werden. Dabei kann sich die Zugabereihenfolge und der Zugabezeitpunkt der Einzelkomponenten entscheidend auf die erhaltenen Mischungseigenschaften auswirken. Üblicherweise kann die so erhaltene Kautschukmischung in einem Innenmischer oder auf einer Walze bei 40-110°C mit den Vernetzungschemikalien versetzt werden und zur sogenannten Rohmischung für die nachfolgenden Prozeßschritte, wie zum Beispiel Formgebung und Vulkanisation, verarbeitet werden.

Die Vulkanisation der erfindungsgemäßen Kautschukmischungen kann bei Temperaturen von 80 bis 200°C, bevorzugt 130 bis 180 °C, gegebenenfalls unter Druck von 10 bis 200 bar erfolgen.

Die erfindungsgemäßen Kautschukmischungen können zur Herstellung von Formkörpern, zum Beispiel für die Herstellung von Luftreifen, Reifenlaufflächen, Kabelmänteln, Schläuchen, Treibriemen, Förderbändern, Walzenbelägen, Reifen, Schuhsohlen, Dichtungsringen, Profilen und Dämpfungselementen verwendet werden.

Ein weiterer Gegenstand der Erfindung sind Formkörper, erhältlich aus der erfindungsgemäßen Kautschukmischung durch Vulkanisation.

Die erfindungsgemässen Kautschukmischungen weisen den Vorteil auf, dass die dynamische Steifigkeit gleich oder erhöht ist (gleiche oder bessere Handlingeigenschaften) und gleichzeitig der Verlustfaktor bei 60°C (Hysterese) erniedrigt ist (niedriger Rollwiderstand) gegenüber einer Kautschukmischung, enthaltend den unmodifizierten Ausgangsruß.

### Beispiel 1 (Referenz):

Herstellung eines Rußes mit organischen Gruppen durch Umsetzung von N-(4-Thiocyanatophenyl)-maleinsäureamid mit Ruß in trockener Mischung.

500g Ruß N 220 und 100g N-(4-Thiocyanatophenyl)-maleinsäureamid werden 5 min in einem Mixer gemischt und anschließend 5 h in einem Muffelofen bei 180° C getempert. Nach Beendigung der Temperung wird der Ruß in zehn Portionen jeweils in 1 l Aceton suspendiert, abgesaugt, mit Aceton gewaschen und getrocknet.

Das Produkt wird nach der Umsetzung durch Mahlen pulverisiert.
Umsatz: 70% (bezogen auf eingesetztes N-(4-Thiocyanatophenyl)-maleinsäureamid)

### Beispiel 2 (Referenz):

Herstellung eines Rußes mit organischen Gruppen durch Umsetzung von N-(4-Thiocyanatophenyl)-maleinsäureamid mit Ruß in Lösung

500 g Ruß N 220 werden in einer Lösung von 34 g Maleinsäureamid des 4-Rhodanoanilins in 10 l Aceton suspendiert. Das Lösungsmittel wird dann abdestilliert. Die verbleibende Ruß-Reagenz-Mischung wird 5 h bei 180°C im Muffelofen getempert. Nach Beendigung der Temperung wird der Ruß in zehn Portionen jeweils in 1 l Aceton suspendiert, abgesaugt, mit Aceton gewaschen und getrocknet.

Das Produkt wird nach der Umsetzung durch Mahlen pulverisiert.
Umsatz: 82% (bezogen auf das eingesetzte N-(4-Thiocyanatophenyl)-maleinsäureamid)

### Beispiel 3 (Referenz):

### Herstellung eines Rußes mit organischen Gruppen durch Umsetzung von N-(4-Thiocyanatophenyl)-maleinsäureamid mit Ruß in Lösung

500 g Ruß N 220 werden in einer Lösung von 67 g Maleinsäureamid des 4-Rhodanoanilins in 10 1 Aceton suspendiert. Das Lösungsmittel wird dann abdestilliert. Die verbleibende Ruß-Reagenz-Mischung wird 5 h bei 180°C im Muffelofen getempert. Nach Beendigung der Temperung wird der Ruß in zehn Portionen jeweils in 1 1 Aceton suspendiert, abgesaugt, mit Aceton gewaschen und getrocknet.

Das Produkt wird nach der Umsetzung durch Mahlen pulverisiert.
Umsatz: 87% (bezogen auf das eingesetzte N-(4-Thiocyanatophenyl)-maleinsäureamid)

### Beispiel 4 (Referenz):

### Herstellung eines Rußes mit organischen Gruppen durch Umsetzung von N-(4-Thiocyanatophenyl)-maleinsäureamid mit Ruß in Lösung

500 g Ruß N 220 werden in einer Lösung von 100 g Maleinsäureamid des 4-Rhodanoanilins in 10 1 Aceton suspendiert. Das Lösungsmittel wird dann abdestilliert. Die verbleibende Ruß-Reagenz-Mischung wird 5 h bei 180°C im Muffelofen getempert. Nach Beendigung der Temperung wird der Ruß in zehn Portionen jeweils in 1 1 Aceton suspendiert, abgesaugt, mit Aceton gewaschen und getrocknet.

Das Produkt wird nach der Umsetzung durch Mahlen pulverisiert.
Umsatz: 70 % (bezogen auf eingesetztes N-(4-Thiocyanatophenyl)-maleinsäureamid)

### Vergleichsbeispiel 1:

Herstellung von OH-oberflächenmodifiziertem Ruß in Lösung gemäß DE 10012783

500 g Ruß N 220 werden in einer Lösung von 100 g Maleinsäureamid des 4-Hydroxyanilins in 10 1 Acetoin suspendiert. Das Lösungsmittel wird dann abdestilliert. Die verbleibende Ruß-Reagenz-Mischung wird 5 h bei 180°C im Muffelofen getempert. Nach Beendigung der Temperung wird der Ruß in zehn Portionen jeweils in 1 1 Aceton suspendiert, abgesaugt, mit Aceton gewaschen und getrocknet.

Das Produkt wird nach der Umsetzung durch Mahlen pulverisiert.
Umsatz: 70 %

### Vergleichsbeispiel 2:

Herstellung von oberflächenmodifiziertem Ruß durch Umsetzung von N-(4-Sulfamoylphenyl)-maleinsäureamid mit Ruß gemäß DE 10012783 in trockener Mischung

500 g N 220 und 70 g N-(4-Sulfamoyl-phenyl)maleinsäureamid werden 5 min in einem Mischer gemischt und anschließend 5 h bei 180°C getempert. Das Produkt wird nach der Umsetzung durch Mahlen pulverisiert.
Umsatz: (bezogen auf eingesetztes N-(4-Sulfamoylphenyl)-maleinsäureamid) 59 %

### Beispiel 5:

### Kautschukmischungen

Die für die Kautschukmischungen verwendete Rezeptur ist in der Tabelle 1 angegeben. Dabei bedeutet die Einheit phr Gewichtsanteile bezogen auf 100 Teile des eingesetzten Rohkautschuks. Das allgemeine Verfahren zur Herstellung von Kautschukmischungen und deren Vulkanisate ist in dem folgenden Buch beschrieben: "Rubber Technology Handbook", W. Hofmann, Hanser Verlag 1994.

**Tabelle 1**

| Substanz | Vergleichsmischung 1 [phr] | Vergleichsmischung 2 [phr] | Mischung 3 [phr] |
|---|---|---|---|
| **1. Stufe** | | | |
| Buna SBR 1500 | 100 | 100 | 100 |
| Ruß N 220 | 60 | - | - |
| Vergleichsbeispiel 1 | - | 60 | - |
| Erfindungsgemäßer Ruß mit organischen | - | - | 60 |
| Gruppen gemäß | | | |
| Beispiel 1 | | | |
| ZnO RS | 3 | 3 | 3 |
| Edenor ST1 GS | 2 | 2 | 2 |
| Protektor G3108 | 1 | 1 | 1 |

| **2. Stufe** | | | |
|---|---|---|---|
| Batch Stufe 1 | | | |

| **3. Stufe** | | | |
|---|---|---|---|
| Batch Stufe 2 | | | |
| Vulkacit CZ | 1,5 | 1, 5 | 1, 5 |
| Schwefel | 1, 5 | 1,5 | 1, 5 |

Bei dem Polymer Buna SBR 1500 handelt es sich um ein in Emulsion polymerisiertes SBR-Copolymer der Firma Buna SOW Leuna Olefinverbund GmbH mit einem Styrolgehalt von 23,5 % und einer Viskosität ML(1+4) 100 °C von 50.

Als Stearinsäure wird Edenor ST1 GS der Firma Caldic Deutschland GmbH verwendet und Protektor G3108 ist ein Wachs der Firma Paramelt B.V.. Vulkacit CZ (CBS) ist ein Handelsprodukt der Bayer AG.

Der Ruß N 220 ist ein ASTM Ruß und wird als Corax N 220 von der Degussa AG hergestellt.

Die Kautschukmischungen werden in einem Innenmischer entsprechend der Mischvorschrift in Tabelle 2 hergestellt.

**Tabelle 2**

| | | **Stufe 1** |
|---|---|---|
| **Einstellungen** | | |
| Mischaggregat | | Rheomix 600P |
| Drehzahl | | 100 min⁻¹ |
| Stempeldruck | | 5,5 bar. |
| Leervolumen | | 0,08 L |
| Füllgrad | | 0,7 |
| Durchflußtemp. | | 90 °C |

| **Mischvorgang** | | |
|---|---|---|
| 0 bis | 1 min | Buna SBR 1500 |
| 1 bis | 2 min | ½ Ruß, Stearinsäure, ZnO |
| 2 bis | 3 min | ½ Ruß, Protector G3108 |
| | 3 min | entlüften |
| 3 bis | 4 min | mischen und ausfahren |
| Batch-Temp. | | 140-155°C |
| Lagerung | | 24 h bei Raumtemperatur |
| | | |

| | | **Stufe 2** |
|---|---|---|
| **Einstellungen** | | |
| Mischaggregat | | wie in Stufe 1 bis auf: |
| Durchflußtemp. | | 100 °C |
| Füllgrad | | 0,68 |
| **Mischvorgang** | | |
| 0 bis | 2 min | Batch Stufe 1 aufbrechen |
| | 2 min | ausfahren |
| Batch-Temp. | | 140-155°C |
| Lagerung | | 4 h bei Raumtemperatur |

| | | **Stufe 3** |
|---|---|---|
| **Einstellungen** | | |
| Mischaggregat | | wie in Stufe 1 bis auf |
| Drehzahl | | 50 min⁻¹ |
| Füllgrad | | 0,66 |
| Durchflußtemp. | | 70 °C |

| **Mischvorgang** | | |
|---|---|---|
| 0 bis | 2 min | Batch Stufe 2, Beschleuniger, Schwefel ausfahren und auf Labormischwalzwerk Fell bilden |
| | 2 min | |
| | | Homogenisieren : |
| | | 3* links, 3* rechts einschneiden und |
| | | 8* bei engem Walzenspalt (1 mm) und |
| | | 3* bei weitem Walzenspalt (3,5 mm) stürzen |
| | | Fell ausziehen. |
| Batch-Temp. | | <110 °C |

In Tabelle 3 sind die Methoden für die Gummitestung zusammengestellt.

**Tabelle 3**

| Physikalische Testung | | Norm/ Bedingungen |
|---|---|---|
| | | |
| Vulkameterprüfung, 165°C | | DIN 53529/3, ISO 6502 |
| | Dmax - Dmin [dNm] | |
| Stab 1, 23°C | | DIN 53504, ISO 37 |
| | Zugfestigkeit [MPa] | |
| | Spannungswerte [MPa] | |
| | Bruchdehnung [%] | |
| Viskoelastische Eigenschaften, | | DIN 53 513, ISO 2856 |
| MTS, 60°C, 16 Hz, | | |
| 50 N Vorkraft und 25 N | | |
| Amplitudenkraft | | |
| | Komplexer Modul E* [MPa] | |
| | Verlustfaktor tan δ [] | |

Die Ergebnisse der Gummitestung sind in Tabelle 4 zusammengestellt.

**Tabelle 4**

| **Ergebnisse** | | Vergleichsmischung 1 | Vergleichsmischung 2 | Mischung 3 |
|---|---|---|---|---|
| Merkmal | Einheit | | | |
| Vulkanisationszeit | [min] | 30 | 30 | 30 |
| Dmin | [dNm] | 3,4 | 4,3 | 4,3 |
| Dmax-Dmin | [dNm] | 23,3 | 23,0 | 26,9 |
| Stab 1 | | | | |
| Zugfestigkeit | [MPa]. | 23,5 | 23,5 | 22,2 |
| Spannungswert 50 % | [Mpa] | 2,3 | 2,1 | 2,8 |
| Spannungswert 100 % | [MPa] | 4,1 | 3,6 | 5,2 |
| Spannungswert 200 % | [Mpa] | 11,9 | 8,6 | 13,0 |
| Spannungswert 300 % | [MPa] | 22,0 | 15,1 | 21,5 |
| Bruchdehnung | [%] | 325 | 450 | 440 |
| MTS | | | | |
| E*, 60 °C | [MPa] | 16,5 | 16, 4 | 18, 7 |
| Verlustfaktor tanδ 60 °C | [-] | 0,233 | 0,218 | 0,207 |

Die erfindungsgemäße Kautschukmischung zeigt einen deutlich höheren Dmax-Dmin Wert gegenüber den Referenzmischungen. Während die erfindungsgemäße Kautschukmischung ein vergleichbares Spannungswert-Niveau bei 300% Dehnung aufweist, ist der Spannungswert bei 100% Dehnung deutlich erhöht gegenüber den Referenzmischungen. Dies erklärt dann auch die erhöhte dynamische Steifigkeit E*. Der tanδ 60°C Wert ist bei der erfindungsgemäßen Kautschukmischung abgesenkt (verbesserter Rollwiderstand).

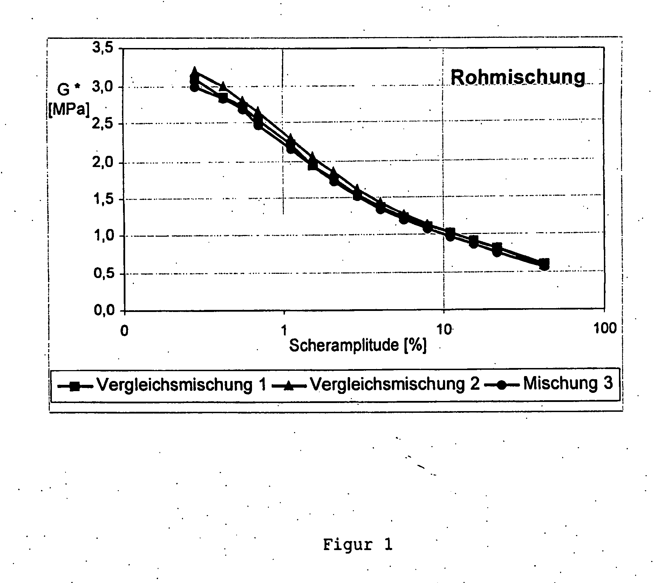

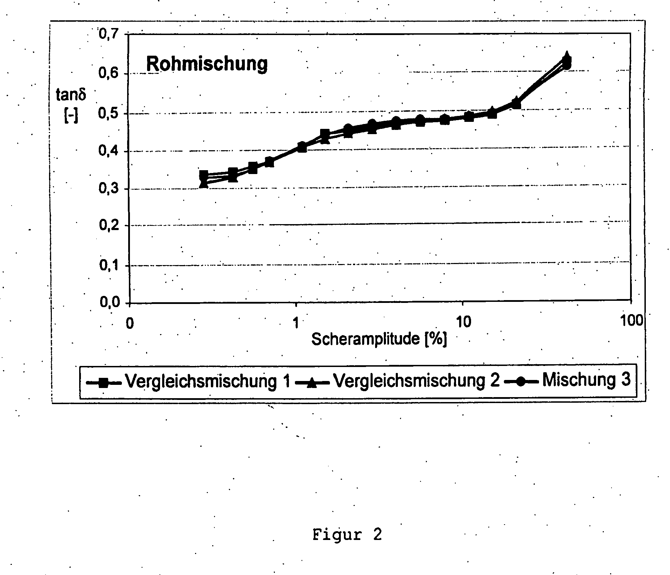

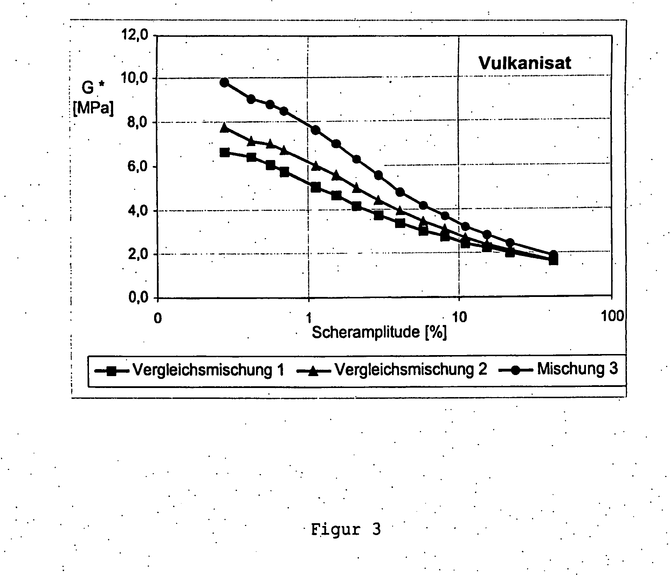

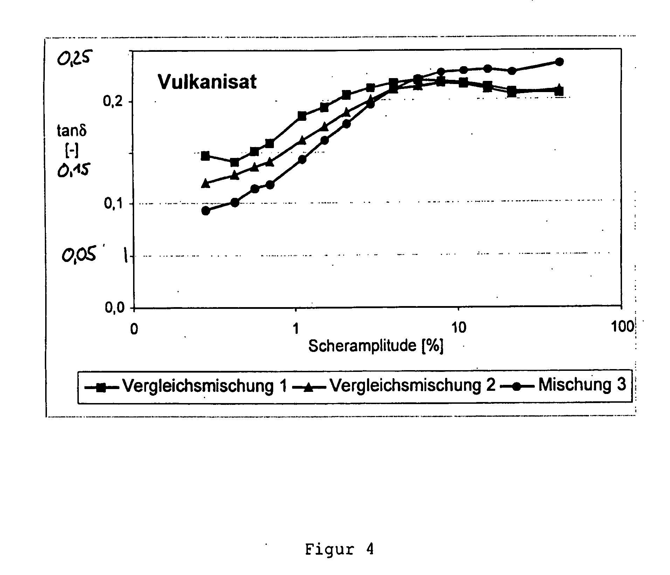

Der Effekt wird bei der RPA-Messung (Figur 1-4) noch deutlicher. Während die Vergleichsmischungen und die erfindungsgemäße Mischung in der Rohmischung (Tabelle 5) identisches Verhalten zeigen , sowohl hinsichtlich G* (Figur 1) als auch tanδ (Figur 2), so ändert sich das Verhalten nach der Vulkanisation (Figur 3+4 / Tabelle 6). Im Vulkanisat zeichnen sich die erfindungsgemäßen Kautschukmischungen dadurch aus, daß sie einen höheren Payne-Effekt und ein niedrigeren tanδ-Wert bei kleinen Auslenkungen haben.

**Tabelle 5**

| Rohmischung | Vergleichsmischung 1 | Vergleichsmischung 2 | Mischung 3 |
|---|---|---|---|
| G*_{RM}@0.28% | 3,1064 | 3,2006 | 2,9951 |
| G*_{RM}@42% | 0,61969 | 0,59456 | 0,58877 |
| Delta G*_{RM} | 2,48671 | 2,60604 | 2,40633 |
| tanδ_{RM}@0.28% | 0,335 | 0,315 | 0,328 |

**Tabelle 6**

| Vulkanisat | Vergleichsmischung 1 | Vergleichsmischung 2 | Mischung 3 |
|---|---|---|---|
| G*v@0.28% | 6,662 | 7,757 | 9,799 |
| G*v@42% | 1,628 | 1,619 | 1,867 |
| Delta G*v | 5,034 | 6,138 | 7,932 |
| tanδᵥ@0.28% | 0,147 | 0,12 | 0,094 |

### Beschreibung der Prüfmethode RPA:

Die RPA Daten werden mit einem Rubber Process Analyzer (RPA) der Firma alpha-technologies gemessen (Deformationsart: Scherung-Torsion, Temperatur: 60°C, Frequenz: 1,6 Hz, Dynamische Scheramplitude: 0,28% - 42%, in aufsteigender Folge 15 Meßpunkte äquidistant auf logarithmischer Skala der Scher-Amplitude). Die Auftragung des dynamischen Schermoduls als Funktion der Amplitude in dem obengenannten Amplitudenbereich beschreibt den Payne-Effekt.

Das Meßprinzip ist in J. Fröhlich and H.D. Luginsland, Rubber World, Vol 224, No. 1, S.28ff beschrieben. Durchführung:
Eine Rohmischungsprobe aus der jeweils letzten Mischstufe (productive step) wird im RPA bei 60°C dem oben aufgeführten Amplitudensweep unterzogen. Als Meßergebnisse erhält man den dynamische Modul G*_{RM} und den Verlustfaktor tand_{RM} als Funktion der Scheramplitude. Als Delta G*_{RM} := G*_{RM}@0.28% - G*_{RM}@42% kann der eigentliche Payne-Effekt berechnet werden. G*_{RM} und tanδ_{Rm} bei unterschiedlichen Amplituden zwischen 0,28% und 42% sowie Delta_G*_{RM}, sind die so bezeichneten RPA-Daten für die Rohmischung.

In einer weiteren Messung wird eine neue Rohmischungsprobe aus der jeweils letzten Mischstufe (productive step) im RPA zunächst bei einer Vulkanisationstemperatur T_vulc für eine Vulkanisationsdauer von t_vulc vulkanisiert. Direkt anschließend wird die so vulkansierte Probe im RPA ohne Öffnen der Meßkammer mit Hilfe von Druckluft (Raumtemperatur) solange abgekühlt, bis die Meßtemperatur von 60°C erreicht und stabilisiert ist. Dieser Vorgang liegt - abhängig von der Vulkanisationstemperatur und der verwendeten Probe - im Bereich von einigen Minuten - typisch ist etwa 2-5 Minuten - und wird vom RPA automatisch durchgeführt. Sofort daran anschließend wird - ohne zwischenzeitliches Öffnen der Meßkammer - diese vulkanisierte Probe im RPA bei 60°C einem oben aufgeführten Amplitudensweep unterzogen. Als Meßergebnisse fallen der dynamische Modul G*ᵥ und der Verlustfaktor tanδᵥ als Funktion der Scheramplitude an. Als Delta G*ᵥ := G*ᵥ@0.28% - G*ᵥ@42% kann dann der eigentliche Payne-Effekt berechnet werden. G*ᵥ und tanδᵥ bei unterschiedlichen Amplituden zwischen 0,28% und 42% sowie Delta_G*ᵥ, sind dann die so bezeichneten RPA-Daten für das Vulkanisat.

### Beispiel 6:

### Kautschukmischungen

Die für die Kautschukmischungen verwendete Rezeptur ist in der Tabelle 7 angegeben.

**Tabelle 7**

| Substanz | Vergleichsmischung 4 [phr] | Mischung 5 [phr] | Mischung 6 [phr] | Mischung 7 [phr] |
|---|---|---|---|---|
| **1. Stufe** | | | | |
| Buna SBR 1500 | 100 | 100 | 100 | 1000 |
| Ruß N 220 | 60 | - | | - |
| Erfindungsgemäßer Ruß gemäß Beispiel 2 | - | 60 | | - |
| Erfindungsgemäßer Ruß gemäß Beispiel 3 | - | - | 60 | - |
| Erfindungsgemäßer Ruß gemäß Beispiel 4 | - | - | - | 60 |
| ZnO RS | 3 | 3 | 3 | 3 |
| Edenor ST1 GS | 2 | 2 | 2 | 2 |
| Protektor G3108 | 1 | 1 | 1 | 1 |

| **2. Stufe** | | | | |
|---|---|---|---|---|
| Batch Stufe 1 | | | | |

| **3. Stufe** | | | | |
|---|---|---|---|---|
| Batch Stufe 2 | | | | |
| Vulkacit CZ | 1,5 | 1,5 | 1,5 | 1,5 |
| Schwefel | 1,5 | 1,5 | 1,5 | 1,5 |

Die Kautschukmischungen werden in einem Innenmischer entsprechend der Mischvorschrift in Tabelle 8 hergestellt.

**Tabelle 8**

| | | **Stufe 1** |
|---|---|---|
| **Einstellungen** | | |
| Mischaggregat | | Brabender 350 S |
| Drehzahl | | 50 min⁻¹ |
| Stempeldruck | | 5,5 bar |
| Leervolumen | | 0, 39 L |
| Füllgrad | | 0,68 |
| Durchflußtemp. | | 90 °C |

| **Mischvorgang** | | |
|---|---|---|
| 0 bis | 1 min | Buna SBR 1500 |
| 1 bis | 2 min | ½ Ruß, Stearinsäure, ZnO |
| 2 bis | 3 min | ½ Ruß, Protector G3108 |
| | 3 min | entlüften |
| 3 bis | 4 min | mischen und ausfahren |
| Batch-Temp. | | 145-155°C |
| Lagerung | | 24 h bei Raumtemperatur |
| | | |

| | | **Stufe 2** |
|---|---|---|
| **Einstellungen** | | |
| Mischaggregat | | wie in Stufe 1 bis auf: |
| Durchflußtemp. | | 100 °C |
| Drehzahl | | 70 min⁻¹ |
| Füllgrad | | 0,66 |

| **Mischvorgang** | | |
|---|---|---|
| 0 bis | 2 min | Batch Stufe 1 aufbrechen |
| | 2 min | ausfahren |
| Batch-Temp. | | 145-155°C |
| Lagerung | | 24 h bei Raumtemperatur |

| | | **Stufe 3** |
|---|---|---|
| **Einstellungen** | | |
| Mischaggregat | | Tröster Walze WNU1 |
| Drehzahl vorne | | 20 min⁻¹ |
| Drehzahl hinten | | 24 min⁻¹ |
| Walzentemperatur vorne | | 40 °C |
| Walzentemperatur hinten | | 50 °C |

| **Mischvorgang** | | |
|---|---|---|
| 0 bis 2 min | | Batch Stufe 2, Fell bilden |
| 2 bis 4 min | | Beschleuniger, Schwefel einmischen |
| 4 bis 6 min | | 3* links, 3* rechts einschneiden und |
| | | 8* bei engem Walzenspalt und |
| | | 3* bei weitem Walzenspalt stürzen |
| | | Fell ausziehen. |
| Batch-Temp. | | <110 °C |

Die Ergbenisse der Gummitestung sind in Tabellen 9 zusammengestellt.

**Tabelle 9**

| **Ergebnisse** | | **Vergleichsmischung 4** | **Mischung 5** | **Mischung 6** | **Mischung 7** |
|---|---|---|---|---|---|
| Merkmal | Einheit | | | | |
| Vulkanisationszeit | [min] | 25 | 25 | 25 | 25 |
| Dmin | [dNm] | 3,2 | 3, 2 | 3,2 | 3, 3. |
| Dmax-Dmin | [dNm] | 23,0 | 24,8 | 25,3 | 25,6 |
| Stab 1 | | | | | |
| Zugfestigkeit | [MPa] | 17,5 | 26,0 | 22,5 | 22,1 |
| Spannungswert 50 % | [Mpa] | 2,2 | 2,6 | 2,6 | 2,5 |
| Spannungswert 100 % | [MPa] | 3, 9 | 5,0 | 4, 8 | 4,6 |
| Spannungswert 200 % | [Mpa] | 11,9 | 14,4 | 13,4 | 11,8 |
| Spannungswert 300 % | [MPa] | | 24,6 | - | 20,5 |
| Bruchdehnung | [%] | 260 | 315 | 290 | 320 |
| MTS | | | | | |
| E*, 60 °C | [MPa] | 16,6 | 18,8 | 18,3 | 17,2 |
| Verlustfaktor tanδ 60 °C | [-] | 0,193 | 0,175 | 0,155 | 0,151 |

Die erfindungsgemäßen Kautschukmischungen zeigen einen deutlich höheren Dmax-Dmin Wert, Spannungswert bei kleinen Dehnungen und dynamischer Modul (E* 60°C) gegenüber der Referenzmischung. Der tanδ 60°C ist abgesenkt (niedriger Rollwiderstand) gegenüber der Referenzmischung.

### Beispiel 7:

### Kautschukmischungen

Die für die Kautschukmischungen verwendete Rezeptur ist in der Tabelle 10 angegeben

**Tabelle 10**

| Substanz | Vergleichsmischung 8 [phr] | Mischung 9 [phr] | Mischung 10 [phr] | Mischung 11 [phr] |
|---|---|---|---|---|
| **1. Stufe** | | | | |
| Buna VSL 5025-1 | 96 | 96 | 96 | 96 |
| Buna CB 24 | 30 | 30 | 30 | 30 |
| Ruß N 22Ö | 80 | - | - | - |
| Erfindungsgemäßer Ruß gemäß Beispiel 2 | - | 80 | - | - |
| Erfindungsgemäßer Ruß gemäß Beispiel 3 | - | - | 80 | - |
| Erfindungsgemäßer Ruß gemäß Beispiel 4 | - | - | - | 80 |
| ZnO RS | 3 | 3 | 3 | 3 |
| Edenor ST1 GS | 2 | 2 | 2 | 2 |
| Naftolen ZD | 10 | 10 | 10 | 10 |
| Vulkanox 4020 | 1,5 | 1,5 | 1, 5 | 1, 5 |
| Protektor G3108 | 1 | 1 | 1 | 1 |

| **2. Stufe** | | | | |
|---|---|---|---|---|
| Batch Stufe 1 | | | | |

| **3. Stufe** | | | | |
|---|---|---|---|---|
| Batch Stufe 2 | | | | |
| Vulkacit CZ | 1,5 | 1, 5 | 1, 5 | 1, 5 |
| Schwefel | 1,5 | 1,5 | 1,5 | 1,5 |

Bei dem Polymer VSL 5025-1 handelt es sich um ein in Lösung polymerisiertes SBR-Copolymer der Bayer AG mit einem Styrolgehalt von 25 Gew.-% und einem Butadiengehalt von 75 Gew.-%. Das Copolymer enthält 37,5 phr Öl und weist eine Mooney-Viskosität (ML 1+4/100°C) von 50 ±4 auf.

Bei dem Polymer Buna CB 24 handelt es sich um ein cis 1,4- Polybutadien (Neodymtyp) der Bayer AG mit einem cis 1,4-Gehalt von 97 %, einem trans 1,4-Gehalt von 2 % und einem 1,2-Gehalt von 1 %.

Als aromatisches Öl wird Naftolen ZD der Chemetall verwendet; bei Vulkanox 4020 handelt es sich um 6PPD der Bayer AG.

Die Kautschukmischungen werden in einem Innenmischer entsprechend der Mischvorschrift in Tabelle 11 hergestellt.

**Tabelle 11**

| | | **Stufe 1** |
|---|---|---|
| **Einstellungen** | | |
| Mischaggregat | | Brabender 350 S |
| Drehzahl | | 80 min⁻¹ |
| Stempeldruck | | 5,5 bar |
| Leervolumen | | 0,39 L |
| Füllgrad | | 0, 68 |
| Durchflußtemp. | | 90 °C |

| **Mischvorgang** | | |
|---|---|---|
| 0 bis | 1 min | Buna VSL 5025-1, Buna CB 24 |
| 1 bis | 3 min | ½ Ruß, Stearinsäure, ZnO, Naftolen ZD |
| 3 bis | 4 min | ½ Ruß, Vulkanox 4020, Protector G3108 |
| | 4 min | säubern |
| 4 bis | 5 min | mischen und ausfahren |
| Batch-Temp. | | 145-155°C |
| Lagerung | | 24 h bei Raumtemperatur |
| | | |

| | | **Stufe 2** |
|---|---|---|
| **Einstellungen** | | |
| Mischaggregat | | wie in Stufe 1 bis auf: |
| Drehzahl | | 95 min⁻¹ |
| Füllgrad | | 0,66 |

| **Mischvorgang** | | |
|---|---|---|
| 0 bis | 2 min | Batch Stufe 1 aufbrechen |
| | 2 min | ausfahren |
| Batch-Temp. | | 145-155°C |
| Lagerung | | 24 h bei Raumtemperatur |

| | | **Stufe 3** |
|---|---|---|
| **Einstellungen** | | |
| Mischaggregat | | Tröster Walze WNU1 |
| Drehzahl vorne | | 20 min⁻¹. |
| Drehzahl hinten | | 24 min⁻¹ |
| Walzentemperatur vorne | | 40°C |
| Walzentemperatur hinten | | 50°C |

| **Mischvorgang** | | |
|---|---|---|
| 0 bis 2 min | | Batch Stufe 2, Fell bilden |
| 2 bis 4 min | | Beschleuniger, Schwefel einmischen |
| 4 bis 6 min | | 3* links, 3* rechts einschneiden und |
| | | 8* bei engem Walzenspalt und |
| | | 3* bei weitem Walzenspalt stürzen |
| | | Fell ausziehen. |
| Batch-Temp. | | <110 °C |

Die Ergbenisse der Gummitestung sind in Tabelle 12 zusammengestellt.

**Tabelle 12**

| **Ergebnisse** | | Vergleichsmischung 8 | Mischung 9 | Mischung 10 | Mischung 11 |
|---|---|---|---|---|---|
| Merkmal | Einheit | | | | |
| Vulkanisationszeit | [min] | 35 | 35 | 35 | 35 |
| Dmin | [dNm] | 2,5 | 3,0 | 3,1 | 2,8 |
| Dmax-Dmin | [dNm] | 13,6 | 18,7 | 19,7 | 15,9 |

| Stab 1 | | | | | |
|---|---|---|---|---|---|
| Zugfestigkeit | (MPa] | 14,8 | 15,2 | 16,6 | 14,9 |
| Spannungswert 50 % | [Mpa] | 1,5 | 1,5 | 1,6 | 1, 5 |
| Spannungswert 100 % | [MPa] | 2,8 | 2,8 | 3,0 | 2,7 |
| Spannungswert 200 % | [Mpa] | 7,2 | 7,3 | 7,9 | 6,7 |
| Spannungswert 300 % | [MPa] | 12,7 | 13,1 | 13,9 | 12,2 |
| Bruchdehnung | **[**%]. | 340 | 335 | 350 | 355 |

| MTS | | | | | |
|---|---|---|---|---|---|
| E*, 60 °C | [MPa] | 10,8 | 16,7 | 17,6 | 14,7 |
| Verlustfaktor tanδ 60 OC | [-] | 0,246 | 0,223 | 0,206 | 0,190 |

Die erfindungsgemäßen Kautschukmischungen zeigen einen deutlich höheren Dmax-Dmin Wert, dynamischer Modul (E* 60°C) und einen höheren oder gleichen Spannungswert bei kleinen Dehnungen gegenüber der Referenzmischung. Der tanδ 60°C ist abgesenkt (niedriger Rollwiderstand) gegenüber der Referenzmischung

### Beispiel 8:

### Kautschukmischungen

Die für die Kautschukmischungen verwendete Rezeptur ist in der Tabelle 13 angegeben

**Tabelle 13**

| Substanz | Vergleichsmischung 12 [phr] | Mischung 13 [phr] | Mischung 14 [phr] | Mischung 15 [phr] |
|---|---|---|---|---|
| **1. Stufe** | | | | |
| Naturkautschuk | 100 | 100 | 100 | 100 |
| Ruß N 220 | 52 | - | - | - |
| Erfindungsgemäßer Ruß gemäß Beispiel 2 | - | 52 | - | - |
| Erfindungsgemäßer Ruß gemäß Beispiel 3 | - | - | 52 | - |
| Erfindungsgemäßer Ruß gemäß Beispiel 4 | - | - | - | 52 |
| ZnO RS | 3 | 3 | 3 | 3 |
| Edenor ST1 GS | 3 | 3 | 3 | 3 |
| Vulkanox HS/LG | 1 | 1 | 1 | 1 |
| Vulkanox 4020 | 1 | 1 | 1 | 1 |
| Protektor G3108 | 1 | 1 | 1 | 1 |

| **2. Stufe** | | | | |
|---|---|---|---|---|
| Batch Stufe 1 | | | | |

| **3. Stufe** | | | | |
|---|---|---|---|---|
| Batch Stufe 2 | | | | |
| Rhenogran TBBS-80 | 1,2 | 1,2 | 1,2 | 1,2 |
| Schwefel | 1,5 | 1,5 | 1,5 | 1, 5 |

Bei dem Naturkautschuk handelt es sich um SMR 10 (aus Malaysia, ML(1+4) 100°C = 60-70).

Bei Vulkanox HS/LG handelt es sich um TMQ der Rhein-Chemie GmbH Mannheim. Rhenogran TBBS-80 ist ein 80 %-N-tert.-Butyl-2-benzothiazolsulfenamid Beschleuniger der Rhein-Chemie GmbH Mannheim.

Die Kautschukmischungen werden in einem Innenmischer entsprechend der Mischvorschrift in Tabelle 14 hergestellt.

**Tabelle 14**

| | | **Stufe 1** |
|---|---|---|
| **Einstellungen** | | |
| Mischaggregat | | Brabender 350 S |
| Drehzahl | | 70 min⁻¹ |
| Stempeldruck | | 5,5 bar |
| Leervolumen | | 0,39 L |
| Füllgrad | | 0,68- |
| Durchflußtemp. | | 90 °C |

| **Mischvorgang** | | |
|---|---|---|
| 0 bis | 1 min | Naturkautschuk |
| 1 bis | 2 min | ½ Ruß, Stearinsäure, ZnO |
| 2 bis | 3 min | /LG, ½ Ruß, Vulkanox 4020, Vulkanox HS/LG, Protector G3108 |
| | 3 min | säubern und entlüften |
| 3 bis | 5 min | mischen und ausfahren (eventuell Drehzahlvariation) |
| Batch-Temp. | | 145-155°C |
| Lagerung | | 24 h bei Raumtemperatur |
| | | |

| | | **Stufe 2** |
|---|---|---|
| **Einstellungen** | | |
| Mischaggregat | | wie in Stufe 1 bis auf: |
| Drehzahl | | 100 min⁻¹ |
| Durchflußtemp. | | 100 °C |
| Füllgrad | | 0,66 |
| **Mischvorgang** | | |
| 0 bis | 2 min | Batch Stufe 1 aufbrechen |
| | 2 min | ausfahren |
| Batch-Temp. | | 145-155°C |
| Lagerung | | 24 h bei Raumtemperatur |

| | | **Stufe 3** |
|---|---|---|
| **Einstellungen** | | |
| Mischaggregat | | Tröster Walze WNU1 |
| Drehzahl vorne | | 20 min⁻¹ |
| Drehzahl hinten | | 24 min⁻¹ |
| Walzentemperatur vorne | | 40 °C |
| Walzentemperatur hinten | | 50 °C |

| **Mischvorgang** | | |
|---|---|---|
| 0 bis 2 min | | Batch Stufe 2, Fell bilden |
| 2 bis 4 min | | Beschleuniger, Schwefel einmischen |
| 4 bis 6 min | | 3* links, 3* rechts einschneiden und |
| | | 8* bei engem Walzenspalt und |
| | | 3* bei weitem Walzenspalt stürzen |
| | | Fell ausziehen. |
| Batch-Temp. | | <110 °C |

Die Ergbenisse der Gummitestung sind in Tabelle 15 zusammengestellt.

**Tabelle 15**

| **Ergebnisse** | | Vergleichsmischung 12 | Mischung 13 | Mischung 14 | Mischung 15 |
|---|---|---|---|---|---|
| Merkmal | Einheit | | | | |
| Vulkanisationszeit | [min] | 25 | 25 | 25 | 25 |
| Dmin | [dNm] | 1,3 | 1,3 | 1,2 | 1,3 |
| Dmax-Dmin | [dNm] | 17,0 | 18,5 | 18,6 | 18,1 |

| Stab 1 | | | | | |
|---|---|---|---|---|---|
| Zugfestigkeit | [MPa] | 25,9 | 27,5 | 24,7 | 24,3 |
| Spannungswert 50 % | [Mpa] | 1,4 | 1,6 | 1,5 | 1,5 |
| Spannungswert 100 % | [MPa] | 2,5 | 3, 0 | 2,7 | 2,7 |
| Spannungswert 200 % | [Mpa] | 7,3 | 7,6 | 6,7 | 6,7 |
| Spannungswert 300 % | [MPa] | 14,0 | 13,8 | 12,3 | 12,1 |
| Bruchdehnung | [%] | 490 | 535 | 515 | 515 |

| MTS | | | | | |
|---|---|---|---|---|---|
| E*, 60 °C | [MPa] | 8,0 | 8,4 | 8,5 | 8,2 |
| Verlustfaktor tan8 60 °C | [-] | 0,144 | 0,126 | 0,124 | 0,125 |

Die erfindungsgemäßen Kautschukmischungen zeigen einen deutlich höheren Dmax-Dmin Wert, dynamischer Modul (E* 60°C) und Spannungswert bei kleinen Dehnungen gegenüber der Referenzmischung. Der tanδ 60°C ist abgesenkt (niedriger Rollwiderstand) gegenüber der Referenzmischung.

## Patentansprüche

1. Kautschukmischung, **dadurch gekennzeichnet, daß** diese einen Kautschuk oder eine Mischung von Kautschuken und einen Ruß mit organischen Gruppen in einer Menge von 10 bis 150 Gew.-Teile, wobei die Gew.-Teile auf 100 Gew.-Teile Kautschuk bezogen sind, enthält, und die organische Gruppe eine N-(4-Thiocyanatalkyl)-succinimid-, N-(4-Thiocyanataryl)-succinimid-, N-(4-Thiocyanatalkyl)-amid- oder N-(4-Thiocyanataryl)-amid- Gruppe ist.

2. Verfahren zur Herstellung von Kautschukmischungen nach Anspruch 1, **dadurch gekennzeichnet, daß** man den Kautschuk oder die Mischung von Kautschuken und den Ruß mit organischen Gruppen in einem Mischaggregat mischt.

3. Verwendung der Kautschukmischung gemäß Anspruch 1 in Luftreifen, Reifenlaufflächen, Kabelmänteln, Schläuchen, Treibriemen, Förderbändern, Walzenbelägen, Reifen, Schuhsohlen, Dichtungsringen, Profilen und Dämpfungselementen.

4. Formkörper, erhältlich aus einer Kautschukmischung nach Anspruch 1, durch Vulkanisation.

5. Kautschukmischung gemäß Anspruch 1, **dadurch gekennzeichnet, dass** die dynamische Steifigkeit und der Verlustfaktor tanδ bei 60°C gegenüber einer Kautschukmischung, enthaltend den unmodifizierten Ausgangsruß, verbessert ist.

## Claims

1. A rubber mixture **characterized in that** it comprises a rubber or a blend of rubbers and a carbon black having organic groups in a quantity of 10 to 150 parts by weight, the parts by weight relating to 100 parts by weight of rubber, and the organic group is an N-(4-thiocyanate alkyl)succinimide, N-(4-thiocyanate aryl)succinimide, N-(4-thiocyanate alkyl)amide or N-(4-thiocyanate aryl)amide group.

2. A process for the production of rubber mixtures according to claim 1, **characterized in that** the rubber or the blend of rubbers and the carbon black having organic groups are mixed in a mixer.

3. Use of the rubber mixture according to claim 1 in pneumatic tyres, tyre treads, cable sheaths, tubes, drive belts, conveyor belts, roll coverings, tyres, shoe soles, sealing rings, profiles and damping elements.

4. A molded article obtainable from a rubber mixture according to claim 1 by vulcanization.

5. The rubber mixture according to claim 1, **characterized in that** the dynamic rigidity and the loss factor tanδ at 60°C are improved in comparison to a rubber mixture containing the unmodified starting carbon black.

## Revendications

1. Mélange de caoutchouc
**caractérisé en ce qu'**
il renferme du caoutchouc ou un mélange de caoutchoucs et un noir de carbone ayant des groupes organiques en quantité de 10 à 150 parties en poids, cette quantité pondérale étant rapportée à 100 parties en poids de caoutchouc, et le groupe organique est un groupe N-(4-thiocyanatalkyl)-succinimide-, N-(4-thiocyanataryle)-succinimide-, N-(4-thiocyanatalkyl)-amide ou N-(4-thiocyanataryl)-amide.

2. Procédé d'obtention d'un mélange de caoutchouc conforme à la revendication 1,
**caractérisé en ce que**
l'on mélange le caoutchouc ou le mélange de caoutchoucs et le noir de carbone comportant des groupes organiques dans un mélangeur.

3. Utilisation du mélange de caoutchouc conforme à la revendication 1, dans des pneumatiques, des bandes de roulement de pneumatiques, des gaines de câbles, des tuyaux, de courroies d'entrainement, des bandes transporteuses, des garnitures de cylindres, des bandages, des semelles de chaussures, des bagues d'étanchéité, des profilés et des éléments d'amortissement.

4. Corps moulé pouvant être obtenu à partir d'un mélange de caoutchouc conforme à la revendication 1 par vulcanisation.

5. Mélange de caoutchouc conforme à la revendication 1,
**caractérisé en ce que**
la rigidité dynamique et le facteur de perte tanδ à 60° sont améliorés par rapport à un mélange de caoutchouc renfermant le noir de carbone de départ non modifié.
